# EUROPEAN PATENT SPECIFICATION

(11) **EP 2 170 819 B1**
(45) Date of publication and mention of the grant of the patent: **21.09.2011**
(21) Application number: 08719314.0
(22) Date of filing: 06.03.2008
(51) Int. Cl.: C07C 405/00, A61K 31/5575, A61K 31/559

(54) **PROSTAGLANDIN PHARMACEUTICAL COMPOSITIONS**
PHARMAZEUTISCHE PROSTAGLANDINZUSAMMENSETZUNGEN
COMPOSITIONS PHARMACEUTIQUES DE PROSTAGLANDINE

(30) Priority: 25.05.2007 IT MI20071073
(43) Date of publication of application: 07.04.2010
(73) Proprietor: CTG Pharma S.r.l., 20131 Milan (IT)
(72) Inventor: DEL SOLDATO, Piero, 20052 Monza (IT); SANTUS, Giancarlo, 20146 Milano (IT); SPARATORE, Anna, 20146 Milan (IT)
(74) Representative: Postiglione, Ferruccio
(86) International application number: PCT/IB2008/000620
(87) International publication number: WO 2008/146105

(56) References cited:
- WO-A-01/74315
- US-B1- 6 476 064
- SCHAAF T K: "N-(METHANESULFONYL)-16-PHENOXYPROSTAGLAND INCARBOXAMIDES : TISSUE- SELECTIVE, UTERINE STIMULANTS" JOURNAL OF MEDICINAL CHEMISTRY, AMERICAN CHEMICAL SOCIETY. WASHINGTON, vol. 24, no. 11, 1 November 1981 (1981-11-01), pages 1353-1359, XP000652663 ISSN: 0022-2623
- DRAGOLI D R ET AL: "Parallel synthesis of Prostaglandin E1 Analogues" JOURNAL OF COMBINATORIAL CHEMISTRY, AMERICAN CHEMICAL SOCIETY, WASHINGTON, US, vol. 1, 1 January 1999 (1999-01-01), pages 534-539, XP002323674 ISSN: 1520-4766
- WANG Y ET AL: "DESIGN AND SYNTHESIS OF 13,14-DIHYDRO PROSTAGLANDIN FIALPHA ANALOGUES AS POTENT AND SELECTIVE LIGANDS FOR THE HUMAN FP RECEPTOR" JOURNAL OF MEDICINAL CHEMISTRY, AMERICAN CHEMICAL SOCIETY. WASHINGTON, vol. 43, no. 5, 1 January 2000 (2000-01-01), pages 945-952, XP000929157 ISSN: 0022-2623

## Description

### Field of the invention

This invention relates to the field of new compounds that are derivatives of prostaglandins whose main biological activity is in the treatment of glaucoma and ocular hypertension.

Glaucoma is a group of diseases of the optic nerve involving loss of retinal ganglion cells in a characteristic pattern of optic neuropathy. Although raised intraocular pressure (IOP) is a significant risk factor for developing glaucoma, there is no set threshold for intraocular pressure that causes glaucoma, a disease that leads to progressive, irreversible loss of vision.

Glaucoma affects 1 in 200 people aged 50 or younger and 1 in 10 over the age of 80: almost 3 million people in the United States and almost 14 million people worldwide have glaucoma, this is the third leading cause of blindness worldwide.

Glaucoma occurs when an imbalance in production and drainage of fluid in the eye (aqueous humour) increases eye pressure to unhealthy levels.

It is known that elevated IOP can be, at least partially, controlled by administering drugs which either reduce the production of aqueous humour within the eye or increase the fluid drainage, such as β-blockers, α-agonists, cholinergic agents, carbonic anhydrase inhibitors or prostaglandin analogs.

However, several side effects are associated with the drugs conventionally used to treat glaucoma. Topical β-blockers show serious pulmonary side effects, depression, fatigue, confusion, impotence, hair loss, heart failure and bradycardia. Topical α-agonists have a fairly high incidence of allergic or toxic reactions; topical cholinergic agents (miotics) can cause visual side effects. The side effects associated with oral carbonic anhydrase inhibitors include fatigue, anorexia, depression, paresthesias and serum electrolyte abnormalities (The Merck Manual of Diagnosis and Therapy, Eighteenth Edition, M. H. Beers and R. Porter Editors, Sec. 9, Ch. 103).

The topical prostaglandin analogs (bimatoprost, latanoprost, travoprost and unoprostone) used in the treatment of glaucoma, can produce ocular side effects, such as increased pigmentation of the iris, ocular irritation, conjunctival hyperaemia, iritis, uveitis and macular oedema (Martindale, Thirty-third edition, p. 1445).

U.S. Patent No. 6,417,228 discloses 13-aza prostaglandins having functional PGF2α receptor agonist activity and their use in treating glaucoma and ocular hypertension.

The application No. WO 90/02553 discloses the use of prostaglandins PGA, PGB, PGE and PGF derivatives, in which the omega chain contains a ring structure for the treatment of glaucoma or ocular hypertension.

It has been also reported (Osborne N.N. et al. Inv. Opthalm & Visual Science 43, 1456-1464, 2002) that the ideal anti-glaucoma drug for the future may be an agent that not only reduces IOP but also possesses neuro-protective (versus retina ganglion cells) and vaso-protective (versus optic nerve head) properties.

It was found that the new compounds object of the present invention possess all these characteristics.

It has been also reported (Hoyng PF, et al. "Topical prostaglandins inhibit trauma-induced inflammation in the rabbit eye". Invest Ophthalmol Vis Sci . 1986 Aug; 27 (8) :1217-25*.*) that pre-treatment with PGE1 and PGF2α led to a lower rise in the aqueous prostaglandin E2 (PGE2) concentration and a reduced inflammatory response after corneal puncture.

It has been suggested that PGE1 and PGF2α reduced the trauma-induced inflammatory response by decreasing the formation of endogenous prostaglandins, as well as reflected by their concentration in aqueous humour.

Nevertheless the use of PGE1 and PGF 2α remains problematic due to their tolerability and side effects. Moreover it remains the need to increase the clinical activity reducing the intraocular pressure of the patients.

### Summary of the invention

Object of the present invention are new derivatives of prostaglandins that release H₂S able not only to eliminate or at least reduce the side effects associated with these compounds, but also to possess an improved pharmacological activity.

It has been surprisingly found that prostaglandin H₂S donating derivatives have a significantly improved overall profile as compared to parent prostaglandins both in terms of wider pharmacological activity and enhanced tolerability.

In particular, it has been found that the prostaglandin H₂S donating derivatives, object of the present invention, can be employed for treating glaucoma and ocular hypertension. The compounds of the present invention are indicated for the reduction of intraocular pressure in patients either with open-angle glaucoma or with chronic angle-closure glaucoma, who underwent peripheral iridotomy or laser iridoplasty.

This invention also relates to processes for preparing these compounds and to pharmaceutical compositions containing these compounds.

In particular the polysulfurated groups, contained in the compounds object of the present invention, linked to the prostaglandins are polysulfurated groups containing 2 or more atoms of sulphur selected from the group comprising organic thiosulfonates or dithiole-thione derivatives such as (5-(p-hydroxyphenyl)-3H-1,2-dithiol-3-thione, or 1,3-dithiol-2-thione-5-carboxylic acid, 3-thioxo-3H-1,2-dithiol-5-carboxylic acid, 3-thioxo-3H-1,2-dithiole-4-carboxylic acid.

### Detailed description of the invention

Object of the present invention are prostaglandin H₂S donating derivatives of general formula (I):

A-Y-W (I)

wherein
A is a residue of prostaglandins or their derivatives of formula (II): 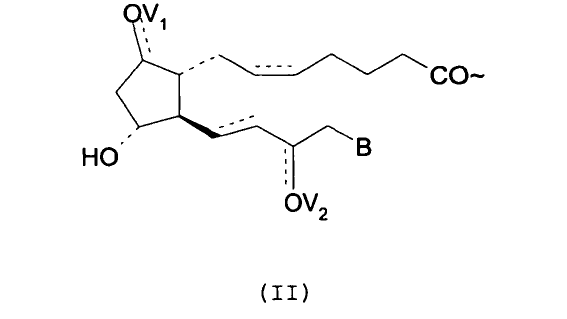 B is - (CH₂)ₘ-CH₃, m is 1-5; or 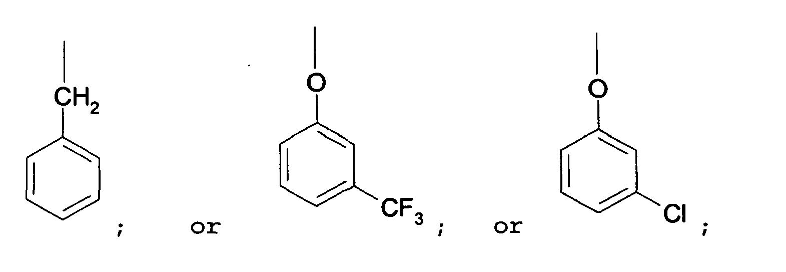 V₁ and V₂ , the same or different to each other, are zero or H;
the bond ---- can be a single bond when V₁ and/or V₂ are H or a double bond;
Y is zero; -(C_{n'})alkyl-, ∼(C_{n'})alkyl-CO-, ∼O-(C_{n'}) alkyl-O-, ∼OOC- (C_{n'}) alkyl-COO-; ~O- (C_{n'}) alkyl-, -HN- (C_{n'}) alkyl-, -OOC- (C_{n'}) alkyl-; ∼(C_{n'}) alkyl-O-CO-(C_{n"})alkyl-; ~(C_{n'})alkyl-CO-O-(C_{n"}) alkyl- wherein (C_{n'})alkyl and (C_{n"}) alkyl are straight or branched, and n' and n", the same or different to each other, are 0-10;
W is a polysulfurated group containing 2 or more atoms of sulphur, selected from the group comprising an organic thiosulfonate moiety or a dithiole-thione derivative:
more in particular, as a further preferred embodiment, W is an organic thiosulfonate moiety having formula (III) :

∼S-SO₂-R (III)

wherein ∼S-SO₂-R is linked to A-Y∼; R is a straight or branched alkyl, such as methyl, ethyl, propyl; alkenyl, alkinyl; alkylaryl, alkenylaryl, alkinylaryl; arylalkyl, arylalkenyl, arylalkinyl; or cycloalkyl, cycloalkenyl, cycloalkinyl; or aromatic and/or heterocyclic ring, all substituted or unsubstituted;
or more in particular, as a further preferred embodiment, W is a dithiole-thione derivative having the following formula (IV): 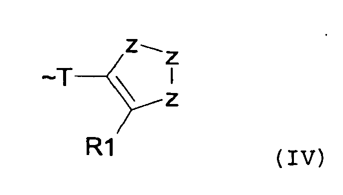 wherein
Z is S (sulphur) and at least 1 Z is C=S (thione) and T is:

-OOC-; or

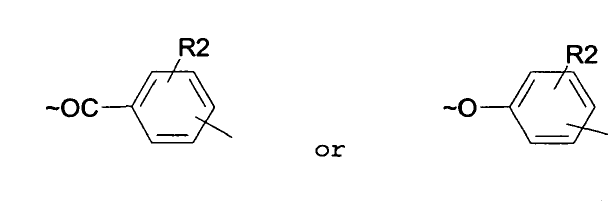

wherein
R1 is H; -COOH; -NH₂; -OH; -SH;
R2 is hydrogen; -COOH; alkyl, alkenyl, alkynyl; aryl; fluoro, chloro, bromo; hydroxyl, alkyloxy, alkenyloxy, aryloxy, acyloxy; amino, alkylamino, arylamino; thio; cyano; nitro; acyl; amido; and a 5 or 6-membered aromatic or non-aromatic ring containing 0, 1, or 2 heteroatoms selected from nitrogen, oxygen, or sulphur;
pharmaceutically acceptable salts and stereoisomers thereof.

As a further preferred embodiment of the compounds of general formula (I) of the present invention (Cₙ)alkyl, (C_{n'})alkyl and (C_{n"})alkyl are (CH₂)_{nA}, (CH₂)_{nA'}, (CH₂)_{nA"} respectively, wherein nA, nA' and nA", the same or different to each other, are 1-10, such as that more preferably Y is selected from the group comprising -(CH₂)_{nA'}-, ~(CH₂)_{nA'}-CO-, ∼O-(CH₂)_{nA'}-O- ,∼OOC-(CH₂)_{nA'}-COO-; ∼O-(CH₂)_{nA'}-, -HN-(CH₂)n_{A'}-, ∼OOC-(CH₂)_{nA'}-; ∼(CH₂)_{nA'}-O-CO-(CH₂)_{nA"}-; ∼(CH₂)_{nA'}-CO-O-(CH₂)_{nA"}- wherein nA, nA' and nA", the same or different to each other, are 1-10.

A further preferred embodiment of the prostaglandin derivative compounds according to the present invention are the compounds of general formula (I) wherein the group -Y-W is selected from the group comprising thiosulfonate moieties derived from the corresponding precursors having formula: S-(2-carboxyethyl)methanthiosulfonate, S-(2-aminoethyl)methanthiosulfonate and S-(2-hydroxyethyl) methanthiosulfonate.

A further preferred embodiment of the prostaglandin derivative compounds according to the present invention, are the compounds of general formula (I) wherein the polysulfurated group W is selected from the group comprising dithiole-thione derivatives of the corresponding precursors having formula: 5-(p-hydroxyphenyl)-3H-1,2-dithiol-3-thione, 1,3-dithiol-2-thione-5-carboxylic acid, 3-thioxo-3H-1,2-dithiole-5-carboxylic acid, 3-thioxo-3H-1,2-dithiole-4-carboxylic acid.

In the present invention the parent compound is considered in its original form or in a proper modification to allow the chemical manipulation with the moiety containing the polysulfurated group.

The prostaglandin or its derivatives, such as the analogs bimatoprost, latanoprost, travoprost and unoprostone, and the moiety containing the polysulfurated group can be linked via different linking groups such as esters, amides, imides, sulfonamides, azo groups, carbamates, carbonates, anhydrides, acetals, thioacetals. The polysulfurated group, i.e. the thiosulfonate moiety or dithiol-thionic derivative, can be also directly linked by an ionic bond to the prostaglandin as salt when Y=0.

Bi-functional linkers (Y), known to the expert in the field, (such as ethyl, propyl, or butyl diols; di-amines; hydroxy amines; etc.) can be optionally present when they are necessary to link the drug (prostaglandin) to the polysulfurated group.

As a further object of the present invention are the preferred compounds according to general formula (I), such as:
(11α,13*E*,15*S*)-11,15-dihydroxy-9-oxoprost-13-en-1-oic acid 4-(3*H*-1,2-dithiole-3-thione-5-yl)-phenyl ester 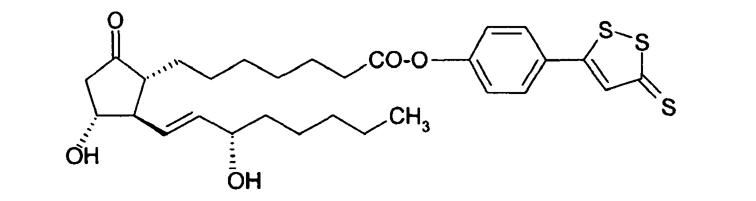
(5*Z*)-7-[(1*R*,2*R*,3*R*,5*S*)-3,5-dihydroxy-2-[(3*R*)-3-hydroxy-5-phenylpentyl]cyclopentyl]-5-heptenoic acid
4-(3*H*-1,2-dithiole-3-thione-5-yl)-phenyl ester 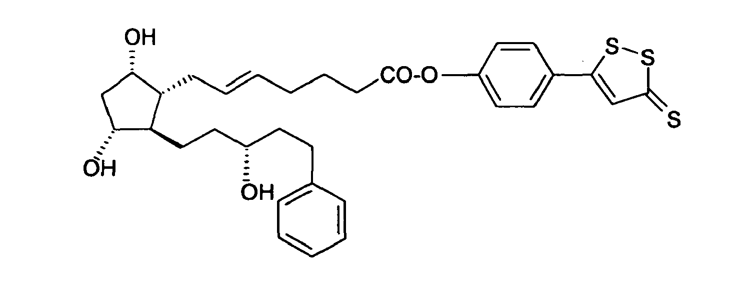
(11α,13*E*,15*S*)-11,15-dihydroxy-9-oxoprost-13-en-1-oic acid 2-(methylsulfonylthio)ethyl ester 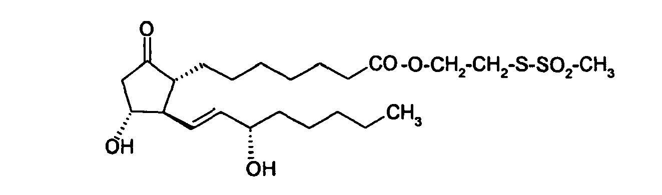
(5*Z*)-7-[(1*R*,2*R*,3*R*,5*S*)-3,5-dihydroxy-2-[(3*R*)-3-hydroxy-5-phenylpentyl]cyclopentyl]-5-heptenoic acid 2-(methylsulfonylthio)ethyl ester 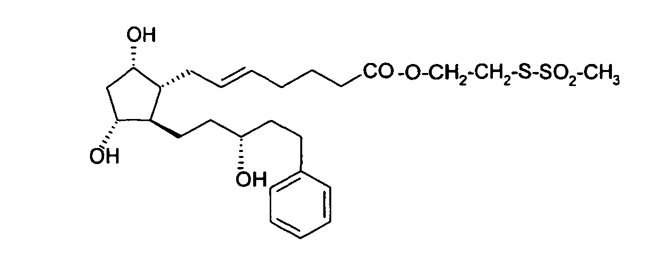
(11α,13*E*,15*S*)-11,15-dihydroxy-9-oxoprost-13-en-1-oic acid 2-(methylsulfonylthio)ethylamide 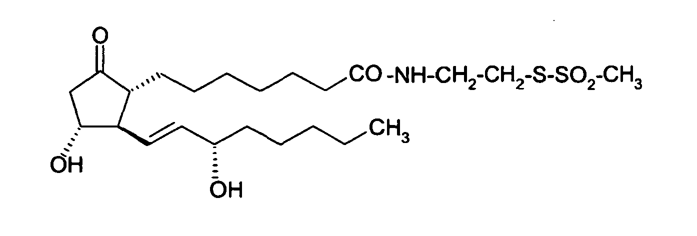
(5*Z*)-7-[(1*R*,2*R*,3*R*,5*S*)-3,5-dihydroxy-2-[3*R*)-3-hydroxy-5-phenylpentyl]cyclopentyl]-5-heptenoic acid 2-(methylsulfonylthio) ethylamide 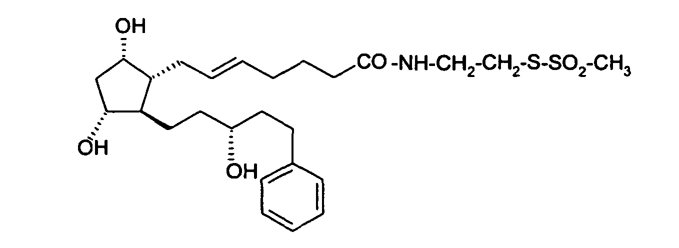
(5*Z*,9α,11α,13*E*,15*S*)-9,11,15-trihydroxy-17-phenyl-18,19,20-trinorprosta-5,13-dienoic acid 2-(methylsulfonylthio) ethylamide

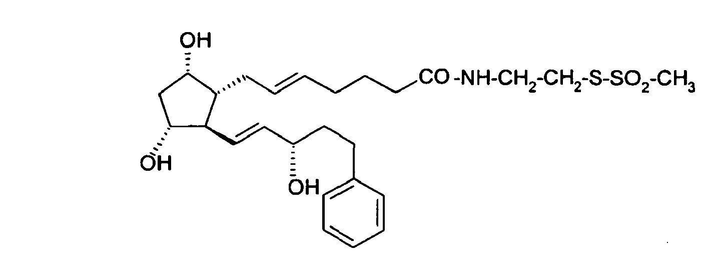

When the compounds include at least one asymmetric carbon atom, the products can be used in racemic mixture or in form of single enantiomer.

It is a further object of the present invention the pharmaceutical acceptable salts of compounds of formula (I), such as for example salts with alkaline metals and alkaline earth metals, non-toxic amines and aminoacids, inorganic acids such as hydrochloric acid, phosphoric acid, etc., or organic acids such as fumaric acid, citric acid, tartaric acid.

Salts of organic thiosulfonates such as, for example, S-(2-carboxyethyl)methanthiosulfonate, S-(2-aminoethyl)methanthiosulfonate with the different prostaglandin derivatives above-described, are also part of the present invention. Salts of dithiolthiones such as, for example, 1,3-dithiol-2-thione-5-carboxylic acid, 3-thioxo-3H-1,2-dithiole-5-carboxylic acid, 3-thioxo-3H-1,2-dithiole-4-carboxylic acid with the different prostaglandin derivatives above-described are also part of the present invention.

According to the present invention it has been found that it is possible to link an organic polysulfurated group to a prostaglandin derivative for treating ocular diseases. The resulting compounds have good bioavailability, increased safety and maintain good efficacy.

The main advantages of the compounds of the present invention are related to their biological activity by topical route.

Further object of the present invention are pharmaceutical compositions comprising at least one compound of the above-said prostaglandin derivative compounds (according to the present invention as for general formula (I) and the preferred compounds as described above) including salts thereof, as an active ingredient, moreover, as a further object of the present invention, in combination with pharmaceutically acceptable adjuvant(s) or carrier(s).

It is a further object of the present invention the use of the prostaglandin derivative compounds as for general formula (I), and the preferred compounds as described above, as a medicament.

A further object of the present invention is the use of compounds according to the present invention, as for general formula (I), and the preferred compounds as described above, for the preparation of a pharmaceutical composition, and therefore the corresponding method for the manufacture of a medicament for preventing, treating or reducing ocular diseases also in combination with other ocular agents.

The prostaglandins derivatives of the present invention can be also used, for example, for treating erectile dysfunction, cerebrovascular and cardiovascular disorders, disorders deriving from peptic ulcer and for inducing abortion.

The compounds of the present invention can be administered in the form of any pharmaceutical formulation, the nature of which will depend upon the route of administration and the nature of the disease to be treated. These pharmaceutical compositions can be prepared by conventional methods, using compatible and pharmaceutically acceptable excipients or vehicles. Examples of such compositions include capsules, tablets, syrups, powders and granulates for the preparation of extemporaneous solutions, injectable preparations, rectal, nasal, ocular, vaginal etc.

A preferred route of administration is the ocular route.

It is a further object of the present invention the process of the synthesis of prostaglandin derivative compounds, as for general formula (I), and preferred compounds as described above, said process comprising the reaction of a prostaglandin or its derivatives with a corresponding precursor of an organic thiosulfonate or of a dithiolthione, moiety W or Y-W, or the reaction of a corresponding precursor of an organic thiosulfonate or of a dithiolthione, moiety W, with a prostaglandin or its derivative, eventually modified with Y, being said W and Y as defined above.

The method for treating glaucoma or ocular hypertension consists in contacting a compound of formula (I) with the eye in order to reduce the eye pressure. The composition contains 0.1-30 µg, and preferably 1-10 µg per application of the active substance. The prostaglandin derivative is mixed with an ophthalmologic compatible vehicle that comprises aqueous solutions, oil solutions, ointments. The vehicle may contain in addition preservatives such as benzalkonium chloride, surfactants like polysorbate 80, liposomes, polymers such as cellulose derivatives, polyinylpyrrolidone, hyaluronic acid that can be used to increase viscosity. It is also possible to use soluble or insoluble insert to administer the drug.

It is a further object of the present invention the use of prostaglandin derivative compounds of general formula (I) and the preferred compounds as described above, for the manufacture of a medicament for preventing, treating or reducing ocular diseases, also in combination with other ocular agents, as well as the method for preventing, treating or reducing ocular diseases, said method comprising the use of prostaglandin derivative compounds of general formula (I) and the preferred compounds as described above.

The following examples further describe the invention.

### EXAMPLE 1. Synthesis of (11α,13E,15S)-11,15-dihydroxy-9-oxoprost-13-en-1-oic acid 4-(3H-1,2-dithiole-3-thione-5-yl)-phenyl ester.

### Step 1: Preparation of 5-(p-hydroxyphenyl)-3H-1,2-dithiol-3-thione.

To 280 mmol of sulphur, 40 mmol of anethole in 20 ml of dimethylacetamide are added. After heating at 145°C for 6 hours, 2.5 g of anethole dithiolethione (ADT) are obtained. The product, washed with ether, was crystallized by ethyl acetate: melting point 110-111°C. Then 1.5 g of ADT are mixed with 7.5 g of pyridine HCl and the mixture is heated for 25 minutes at 215°C. After cooling, 1N HCl in excess is added and the precipitate is filtered, washed and crystallized from ethanol. The obtained compound, 5-(p-hydroxyphenyl)-3H-1,2-ditiol-3-thione, melts at 191-192°C.

### Step 2:

25 mg of the compound prepared in step 1 (0.11 mmol) and catalytic amount of 4-dimethylaminopyridine (DMAP) are added to a solution of (11α,13*E*,15*S*)-11,15-dihydroxy-9-oxoprost-13-en-1-oic acid (PGE1 0.055 mmol; 20 mg) in 1 ml of anhydrous tetrahydrofuran (THF) stirring under nitrogen at a temperature of 0°C. After few minutes 1-(3-dimethylaminoisopropyl)-3-ethyl-carbodiimide hydrochloride (EDAC, 0.08 mmol; 16 mg) is added and the reaction is stirred at room temperature for 15 hours. After evaporation of THF, the residue is dissolved in chloroform and washed with water. The chloroformic solution is dried on anhydrous sodium sulphate, evaporated to dryness and the product is chromatographed on column of silica gel eluting with ethylacetate. The obtained product is red and after washing with ether, has a melting point of 101-105° C.

### EXAMPLE 2. Synthesis of (5Z)-7-[(1R,2R,3R,5S)-3,5-dihydroxy-2-[(3R)-3-hydroxy-5-phenylpentyl] cyclopentyl]-5-heptenoic acid 4-(3H-1,2-dithiole-3-thione-5-yl)-phenyl ester.

39 mg of the compound prepared in Example 1 step 1 (0.17 mmol) and catalytic amount of 4-dimethylaminopyridine (DMAP) are added to a solution of (5*Z*)-7-[(1*R*,2*R*,3*R*,5*S*)-3,5-dihydroxy-2-[(3*R*)-3-hydroxy-5-phenylpentyl]cyclopentyl]-5-heptenoic acid (latanoprost acid 0.087 mmol; 34 mg) in 1 ml of anhydrous tetrahydrofuran (THF) stirring under nitrogen at a temperature of 0°C. After few minutes 1-(3-dimethylaminoisopropyl)-3-ethyl-carbodiimide hydrochloride (EDAC, 0.13 mmol; 25 mg) is added and the reaction is stirred at room temperature for 15 hours. After evaporation of THF, the residue is dissolved in chloroform and washed with water. The chloroformic solution is dried on anhydrous sodium sulphate, evaporated to dryness and the product is chromatographed on column of silica gel with ethylacetate.

After washing with ether the obtained red-coloured product, has a melting point of 91.1-92.2° C.

### EXAMPLE 3. Synthesis of (11α,13E,15S)-11,15-dihydroxy-9-oxoprost-13-en-1-oic acid 2-(methylsulfonylthio)ethyl ester.

*Step 1:* Synthesis of methanethiosulfonic acid S-(2-hydroxyethyl) ester.

A solution of CH₃SO₂Cl (5.9 g) in ethanol (9.2 ml) is added dropwise to a refrigerated (-15°C) solution of Na₂S (46.98 mmol) in ethanol (34.5 ml).

The reaction mixture is stirred at room temperature for 12 hours. After filtration and crystallization from ethanol, sodium methanthiosulfonate, as a white solid, is obtained. The sodium methanthiosulfonate (2.5 g; 18.64 mmol) is dissolved in 30 ml of ethanol and a solution of 2-bromoethanol (2.6 ml; 37.28 mmol) in ethanol (6 ml) is added dropwise. The solution is heated at 100°C for 8 hours under nitrogen. The mixture is filtered, the solution is evaporated to dryness and the residue is dissolved in CHCl₃ and extracted with water.

The aqueous solution is evaporated to dryness, tetrahydrofuran (THF) is added to the residue and the obtained suspension is filtered. The THF solution is evaporated and methanethiosulfonic acid S-(2-hydroxyethyl) ester, as an oily yellow product, is obtained.

### Step 2:

22 mg of the compound prepared in step 1 (0.14 mmol) and catalytic amount of 4-dimethylaminopyridine (DMAP) are added to a solution of (11α,13*E*,15*S*) - 11,15-dihydroxy-9-oxoprost-13-en-1-oic acid (PGE1 0.07 mmol; 25 mg) in 1 ml of anhydrous tetrahydrofuran (THF) stirring under nitrogen at a temperature of 0°C. After few minutes 1-(3-dimethylaminoisopropyl)-3-ethyl-carbodiimide hydrochloride (EDAC, 0.10 mmol; 19.4 mg) is added and the reaction mixture is stirred at room temperature for 15 hours. After evaporation of THF, the residue is dissolved in chloroform and washed with water. The chloroformic solution is dried on anhydrous sodium sulphate, evaporated to dryness and the product is chromatographed on column of silica gel eluting with a mixture of ethylacetate/cyclohexane (80/20) After washing with ether the obtained red-coloured product, has a melting point of 56-58 °C.

### EXAMPLE 4. Synthesis of (5Z)-7-[(1R,2R,3R,5S)-3,5-dihydroxy-2-[(3R)-3-hydroxy-5-phenylpentyl] cyclopentyl]-5-heptenoic acid 2-(methylsulfonylthio) ethyl ester.

In the same manner as described in Example 3 the (5*Z*)-7-[(1*R*,2*R*,3*R*,5*S*)-3,5-dihydroxy-2-[(3*R*)-3-hydroxy-5-phenylpentyl]cyclopentyl]-5-heptenoic acid 2-(methylsulfonylthio)ethyl ester is prepared.

### EXAMPLE 5. Synthesis of (11α,13E,15S)-11,15-dihydroxy-9-oxoprost-13-en-1-oic acid 2-(methylsulfonylthio) ethylamide.

### Step 1: Synthesis of S-(2-aminoethyl) methanthiosulfonate

A solution of CH₃SO₂Cl (5.9 g) in ethanol (9.2 ml) is added dropwise to a refrigerated (-15°C) solution of Na₂S (46.98 mmol) in ethanol (34.5 ml).

The reaction mixture is stirred at room temperature for 12 hours. After filtration and crystallization from ethanol, sodium methanthiosulfonate, as a white solid, is obtained.

The sodium methanthiosulfonate (1.20 g; 8.9 mmol) is dissolved in 17 ml of ethanol and 2-bromoethylamine hydrobromide (1.8 g; 8.9 mmol) is added. The solution is heated at 100°C for 5 hours under nitrogen. At the end of the reaction the mixture is cooled at 0°C filtered to remove NaBr, and the solution is evaporated to obtain an oil that after treatment with ethanol crystallizes and gives a compound with a melting point of 112.0-112.8 °C.

### Step 2:

33 mg of the compound prepared in step 1 (0.14 mmol)and catalytic amount of 4-dimethylaminopyridine (DMAP) are added to a solution of (11α,13*E*,15*S*)-11,15-dihydroxy-9-oxoprost-13-en-1-oic acid (PGE1 0.07 mmol; 25 mg) in CH₂Cl₂ stirring under nitrogen at a temperature of 0°C. After few minutes 1-(3-dimethylaminoisopropyl)-3-ethyl-carbodiimide hydrochloride (EDAC, 0.1 mmol; 19.4 mg) is added and the reaction is stirred at room temperature for 24 hours. After washing with water, 0.1 N HCl, water, NaHCO₃ in a separator funnel, the solution is dried on anhydrous sodium sulphate, filtered and evaporated to dryness. The product is then chromatographed on column of silica gel eluting with ethylacetate/methanol (99.5:0.5). The obtained product has the following NMR: 1H NMR (CDCl₃): δ 6.00 (m, 1H); 5.70-5.45 (m, 2H); 4.10-3.95 (m, 2H); 3.60-3.45 (m, 2H); 3.30 (s, 3H); 3.25 (t, 2H); 2.75-2.60 (m, 1H); 2.40-1.10 (m, 24H); 0.9-0.70 (m, 3H).

### EXAMPLE 6 Synthesis of (5Z)-7-[(1R,2R,3R,5S)-3,5-dihydroxy-2-[(3R)-3-hydroxy-5-phenylpentyl] cyclopentyl]-5-heptenoic acid 2-(methylsulfonylthio) ethylamide.

42 mg of the compound prepared in Example 5 step 1 (0.18 mmol) and catalytic amount of 4-dimethylaminopyridine (DMAP) are added to a solution of (5*Z*)-7-[(1*R*,2*R*,3*R*,5*S*)-3,5-dihydroxy-2-[(3*R*)-3-hydroxy-5-phenylpentyl]cyclopentyl]-5-heptenoic acid (latanoprost acid) (0.09 mmol; 35 mg) in anhydrous THF, stirring under nitrogen at a temperature of 0°C. After few minutes 1-(3-dimethylaminoisopropyl)-3-ethyl-carbodiimide hydrochloride (EDAC, 0.14 mmol; 26 mg) is added and the reaction is stirred at room temperature for 24 hours. After evaporation of THF, the residue is dissolved in CH₂Cl₂ and the solution is washed first with water and then with 0.1 N HCl, water and finally with a sol. of NaHCO₃. The organic solution is dried on anhydrous sodium sulphate, filtered and evaporated to dryness, to obtain a product which is chromatographed on a column of silica gel with ethylacetate. The obtained compound has the following ¹H NMR (CDCl₃): δ 7,35-7.10 (m, 5H) ; 6.55 (s, 1H); 5.50-5.30 (m, 2H); 4.20 (s, 1H); 3.96 (s, 1H); 3.75-3.50 (m, 3H); 3.30 (s, 3H); 3.25 (t, 2H); 2.85-2.60 (m, 2H); 2.45-1.25 (m, 18 H).

### EXAMPLE 7. Synthesis of (5Z,9α,11α,13E,15S)-9,11,15-trihydroxy-17-phenyl-18,19,20-trinorprosta-5,13-dienoic acid 2-(methylsulfonylthio) ethylamide

In the same manner as described in Example 6 the (5*Z*,9α,11α,13*E*,15*S*)-9,11,15-trihydroxy-17-phenyl-18,19,20-trinorprosta-5,13-dienoic acid 2-(methylsulfonylthio) ethylamide is prepared.

### EXAMPLE 8. Biological activity

### Studies on rat retina

The method described by Osborne N.N. et al. (2002) previously reported was used. Briefly, two groups of 16 Wistar rats (200-250 g) received either 5 µl doses of topical test compound (2% in 50% polyethylene glycol-PEG) or 5 µl of vehicle bilaterally twice a day for 2 days. On the third day N-methyl-D-aspartate(NMDA) (5 µl in sterile water) was injected intra-vitreally into a single eye of each animal. The other eye was injected with sterile water. The animals were treated then with test compound or vehicle for 7 days. The Thy-1 antigen is associated with ganglion cells and intraocular injections of NMDA cause a loss of Thy-1 mRNA. The rats were then killed and the retinas removed for mRNA analysis for Thy-1 antigen. Results are expressed as % value to the Thy-1 mRNA levels (relative to rhodopsin) of the treated groups, taking PEG value as 100.

### Studies of IOP (intraocular pressure) on rabbit

The method by Osborne N.N. et al.(2002) previously reported was used. Briefly, two groups of adult male New Zealand albino rabbits weighing 3-3.5 kg were used in the experiments. IOP was measured using a properly calibrated tonometer immediately after topical application of 1 drop of 0.4% benoxinate hydrochloride. The animals received a topical application of 2% of test compound or PEG and measures taken 60 minutes after. Results are expressed as % value to the IOP mmHg values of the treatment groups, taking PEG value as 100. Results that are reported in the table below (Table 1) show that the test compound markedly affects both intraocular pressure and Thy-1 mRNA loss, indicating relevant intraocular hypotensive and neuroprotective properties.

**Table 1**

| Treatment | NMDA effects (%) | IOP values (%) |
|---|---|---|
| Solvent (PEG) | 100 | 100 |
| Compound Example 2 | 52 | 85 |

## Claims

1. Compounds of general formula (I):
A-Y-W (I)
wherein
A is a residue of prostaglandins or their derivatives of formula (II): 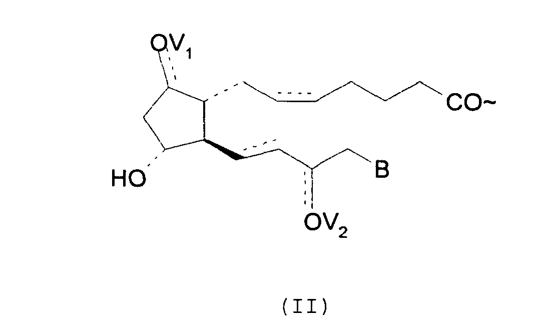 B is -(CH₂)ₘ-CH₃, m is 1-5; or 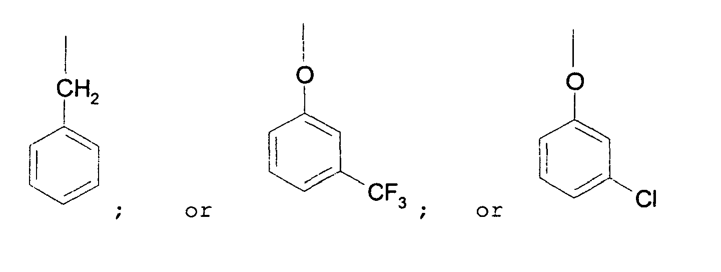 V₁ and V₂, the same or different to each other, are zero or H;
the bond ---- is a single bond when V₁ and/or V₂ are H or a double bond;
Y is zero; -(C_{n'})alkyl-, ~(C_{n'})alkyl-CO-, ∼O-(C_{n'})alkyl-O-, ∼OOC-(C_{n'})alkyl-COO-; ∼O-(C_{n'}) alkyl-, ~HN-(C_{n'})alkyl-, ∼OOC-(C_{n'})alkyl-; ~(C_{n'})alkyl-O-CO-(C_{n"})alkyl-; ∼(C_{n'}) alkyl-CO-O- (C_{n"}) alkyl- wherein (C_{n'})alkyl and (C_{n"})alkyl are straight or branched, and n' and n", the same or different to each other, are 0-10;
W is a polysulfurated group containing 2 or more atoms of sulphur, selected from the group comprising an organic thiosulfonate moiety or a dithiole-thione derivative,
their stereoisomers and salts thereof.

2. Compounds of general formula (I) according to claim 1, wherein W is an organic thiosulfonate moiety having formula:
∼S-SO₂-R (III)
wherein ∼S-SO₂-R is linked to A-Y∼; R is a straight or branched alkyl, selected from the group comprising methyl, ethyl, propyl; alkenyl, alkinyl; alkylaryl, alkenylaryl, alkinylaryl; arylalkyl, arylalkenyl, arylalkinyl or cycloalkyl, cycloalkenyl, cycloalkinyl, or aromatic and/or heterocyclic ring,
all substituted or unsubstituted.

3. Compounds of general formula (I) according to claim 1, wherein W is a dithiole-thione derivative having formula: 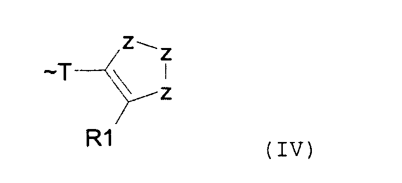 wherein
Z is S (sulphur) and at least 1 Z is a thione C=S; and
T is:
∼OOC-; or 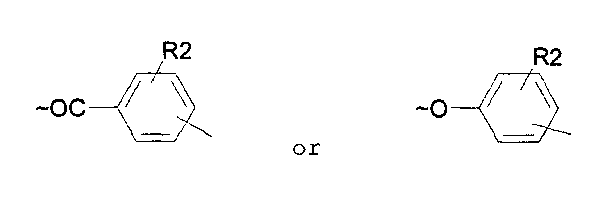
wherein
R1 is H; -COOH; -NH₂; -OH; -SH;
R2 is hydrogen; -COOH; alkyl, alkenyl, alkynyl; aryl; fluoro, chloro, bromo; hydroxyl, alkyloxy, alkenyloxy, aryloxy, acyloxy; amino, alkylamino, arylamino; thio; cyano; nitro; acyl; amido; and a 5 or 6-membered aromatic or non-aromatic ring containing 0, 1, or 2 heteroatoms selected from nitrogen, oxygen, or sulphur.

4. Salts of compounds of general formula (I) according to claim 1, wherein said salts are pharmaceutical acceptable salts of compounds of formula (I).

5. Salts according to claim 4, said salts comprising salts of compounds of formula (I) with: alkaline metals and alkaline earth metals, non-toxic amines and aminoacids, inorganic acids comprising hydrochloric acid, phosphoric acid or organic acids comprising fumaric acid, citric acid, tartaric acid.

6. Compounds of general formula (I) according to claim 2, wherein the group -Y-W is selected from the group comprising thiosulfonate moieties derived from the corresponding precursors having formula: S-(2-carboxyethyl)methanthiosulfonate, S-(2-aminoethyl)methanthiosulfonate and S-(2-hydroxyethyl) methanthiosulfonate.

7. Compounds of general formula (I) according to claim 3, wherein the polysulfurated group W is selected from the group comprising dithiole-thione derivatives of the corresponding precursors having formula: 5-(p-hydroxyphenyl)-3H-1,2-dithiol-3-thione, 1,3-dithiol-2-thione-5-carboxylic acid, 3-thioxo-3H-1,2-dithiole-5-carboxylic acid, 3-thioxo-3H-1,2-dithiole-4-carboxylic acid.

8. Compound of general formula (I) according to claim 1, wherein said compound is selected from the group comprising: (11α,13*E*,15*S*)-11,15-dihydroxy-9-oxoprost-13-en-1-oic acid 4-(3*H*-1,2-dithiole-3-thione-5-yl)-phenyl ester, (5*Z*)-7-[(1*R*,2*R*,3*R*,5*S*)-3,5-dihydroxy-2-[(3*R*)-3-hydroxy-5-phenylpentyl]cyclopentyl]-5-heptenoic acid 4-(3*H*-1,2-dithiole-3-thione-5-yl)-phenyl ester, (11α,13*E*,15*S*)-11,15-dihydroxy-9-oxoprost-13-en-1-oic acid 2-(methylsulfonylthio)ethyl ester, (5*Z*)-7-[(1*R*,2*R*,3*R*,5*S*)-3,5-dihydroxy-2-[(3*R*)-3-hydroxy-5-phenylpentyl]cyclopentyl]-5-heptenoic acid 2-(methylsulfonylthio)ethyl ester, (11α,13*E*,15*S*)-11,15-dihydroxy-9-oxoprost-13-en-1-oic acid 2-(methylsulfonylthio)ethylamide, (5*Z*)-7-[(1*R*,2*R*,3*R*,5*S*)-3,5-dihydroxy-2-[(3*R*)-3-hydroxy-5-phenylpentyl]cyclopentyl]-5-heptenoic acid 2-(methylsulfonylthio)ethylamide, (5*Z*,9α,11α,13*E*,15*S*)-9,11,15-trihydroxy-17-phenyl-18,19,20-trinorprosta-5,13-dienoic acid 2-(methylsulfonylthio) ethylamide.

9. Pharmaceutical composition comprising at least a compound of general formula (I) according to claims 1-8 as an active ingredient and eventually one or more pharmaceutically acceptable adjuvant(s) or carrier(s).

10. Compound of general formula (I) according to claims 1-8 for use as a medicament.

11. Use of a compound of general formula (I) according to claims 1-8 for the manufacture of a medicament for prevention, treatment of ocular diseases.

12. Use of a compound of general formula (I) according to claims 1-8 for the manufacture of a medicament for treatment of ocular diseases, in combination with other ophthalmic agents.

13. Process for the synthesis of compounds of general formula (I) according to claim 1-8, comprising at least a reaction between the corresponding precursor of an organic thiosulfonate or dithiol-thione, moiety W or Y-W, and a prostaglandin compound or at least a reaction between the corresponding precursor of an organic thiosulfonate or dithiol-thione, moiety W and a corresponding precursor of a prostaglandin modified with Y, wherein W and Y have the meaning according to claim 1.

## Patentansprüche

1. Verbindungen mit der allgemeinen Formel (I):
A-Y-W (I),
wobei
A ein Rest von Prostaglandinen oder ihren Derivaten mit der Formel (II): 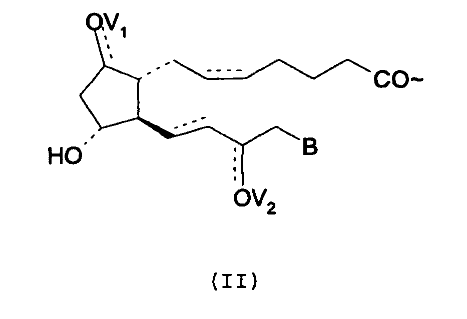 ist,
B gleich -(CH₂)ₘ- CH₃ ist, m gleich 1-5 ist; oder 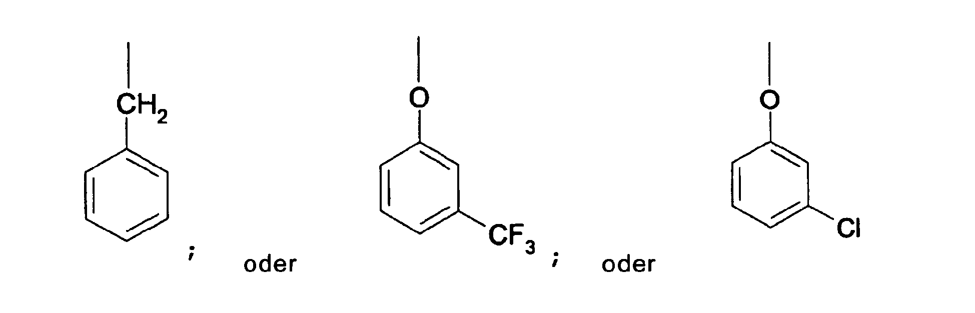 V₁ und V₂ gleich oder voneinander unterschiedlich, Null oder H sind; die Bindung ··· eine Einfachbindung ist, wenn V₁ und/oder V₂ gleich H sind, oder eine Doppelbindung;
Y gleich Null; -(C_{n'})alkyl-, ~(C_{n'})alkyl-CO-, ~O-(C_{n'})alkyl-O-, ~OOC-(C_{n'})alkyl-COO-; ∼O-(C_{n'})alkyl-, ∼HN-(C_{n'})alkyl-, ∼OOC-(C_{n'})alkyl-; ∼(C_{n'})alkyl-O-CO-(C_{n"})alkyl-; ∼(C_{n'})alkyl-CO-O-(C_{n"})alkyl- ist, wobei (C_{n'})alkyl und (C_{n"})alkyl geradkettig oder verzweigt sind und n' und n", gleich oder voneinander unterschiedlich, gleich 0-10 sind;
W eine polysulfurierte Gruppe ist, welche 2 oder mehr Schwefelatome enthält und welche aus der Gruppe ausgewählt wird, welche eine organische Thiosulfonat-Gruppe oder ein Dithiol-Thion-Derivat, ihre Stereoisomere und Salze davon umfasst.

2. Verbindungen mit der allgemeinen Formel (I) gemäß Anspruch 1, wobei W eine organische Thiosulfonat-Gruppe mit der Formel:
∼S-SO₂-R (III)
ist, wobei ∼S-SO₂-R mit A-Y∼ verbunden ist; R ein geradkettiges oder verzweigtes Alkyl ist, welches aus der Gruppe ausgewählt wird, welche Methyl-, Ethyl-, Propyl-; Alkenyl-, Alkinyl-; Alkylaryl-, Alkenylaryl-, Alkinylaryl-; Arylalkyl-, Arylalkenyl-, Arylalkinyl- oder Cycloalkyl-, Cycloalkenyl-, Cycloalkinyl-Gruppen oder einen aromatischen und/oder heterozyklischen Ring, alle substituiert oder unsubstituiert, umfasst.

3. Verbindungen mit der allgemeinen Formel (I) gemäß Anspruch 1, wobei W ein Dithiol-Thion-Derivat mit der Formel 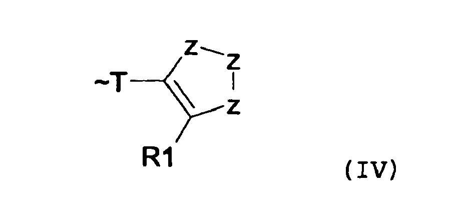 ist, wobei
Z gleich S (Schwefel) ist und mindestens 1 Z ein C=S-Thion ist; und T gleich:
~OOC- ist; oder 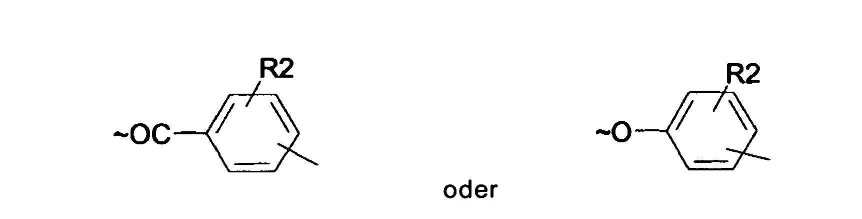 wobei
R1 gleich H; -COOH; -NH₂; -OH; -SH ist;
R2 gleich Wasserstoff; -COOH; eine Alkyl-, Alkenyl-, Alkynyl-; Aryl-;
Fluor-, Chlor-, Brom-; Hydroxyl-, Alkyloxy-, Alkenyloxy-, Aryloxy-, Acyloxy-; Amino-, Alkylamino-, Arylamino-; Thio-; Cyano-; Nitro-; Acyl-; Amido-Gruppe ist; und ein 5- oder 6-gliedriger aromatischer oder nichtaromatischer Ring, welcher 0, 1 oder 2 aus der aus Stickstoff, Sauerstoff und Schwefel bestehenden Gruppe ausgewählte Heteroatome enthält.

4. Salze von Verbindungen mit der allgemeinen Formel (I) nach Anspruch 1, wobei die genannten Salze pharmazeutisch akzeptable Salze von Verbindungen mit der Formel (I) sind.

5. Salze nach Anspruch 4, wobei die genannten Salze Salze von Verbindungen mit der Formel (I) mit: Alkalimetallen und Erdalkalimetallen, nicht-toxischen Aminen und Aminosäuren, anorganischen Säuren, darunter Salzsäure, Phosphorsäure, oder organischen Säuren, darunter Fumarsäure, Zitronensäure, Weinsäure, umfassen.

6. Verbindungen mit der allgemeinen Formel (I) nach Anspruch 2, wobei die Gruppe -Y-W aus der Gruppe ausgewählt wird, welche Thiosulfonat-Gruppen umfasst, die von den entsprechenden Ausgangsstoffen mit der Formel S-(2-Carboxyethyl)methanthiosulfonat, S-(2-Aminoethyl)methanthiosulfonat und S-(2-Hydroxyethyl)methanthiosulfonat abgeleitet sind.

7. Verbindungen mit der allgemeinen Formel (I) nach Anspruch 3, wobei die polysulfurierte Gruppe W aus der Gruppe ausgewählt wird, welche Dithiol-Thion-Derivate der entsprechenden Ausgangsstoffe mit der Formel: 5-(p-Hydroxyphenyl)-3H-1,2-dithiol-3-thion, 1,3-Dithiol-2-thion-5-carboxylsäure, 3-Thioxo-3H-1,2-dithiol-5-carboxylsäure, 3-Thioxo-3H-1,2-dithiol-4-carboxylsäure umfasst.

8. Verbindung mit der allgemeinen Formel (I) nach Anspruch 1, wobei die genannte Verbindung aus der Gruppe ausgewählt wird, welche (11α,13*E*,15*S*)-11,15-Dihydroxy-9-oxoprost-13-en-1-säure-4-(3*H*-1,2-dithiol-3-thion-5-yl)-phenylester, (5*Z*)-7-[(1*R*,2*R*,3*R*,5*S*)-3,5-dihydroxy-2-[(3*R*)-3-hydroxy-5-phenylpentyl]cyclopentyl]-5-heptensäure-4-(3*H*-1,2-dithiol-3-thion-5-yl)-phenylester, (11α,13*E*,15*S*)-11,15-dihydroxy-9-oxoprost-13-en-1-säure-2-(methylsulfonylthio)ethylester, (5*Z*)-7-[(1*R*,2*R*,3*R*,5*S*)-3,5-dihydroxy-2-[(3*R*)-3-hydroxy-5-phenylpentyl]cyclopentyl]-5-heptensäure-2-(methylsulfonylthio)ethylester, (11α,13*E*,15*S*)-11,15-dihydroxy-9-oxoprost-13-en-1-säure-2-(methylsulfonylthio)ethylamid, (5*Z*)-7-[(1*R*,2*R*,3*R*,5*S*)-3,5-dihydroxy-2-[(3*R*)-3-hydroxy-5-phenylpentyl)cyclopentyl]-5-heptensäure-2-(methylsulfonylthio)ethylamid, (5*Z*,9α,11α,13*E*,15*S*)-9,11,15-trihydroxy-17-phenyl-18,19,20-trinorprosta-5,13-diensäure-2-(methylsulfonylthio)ethylamid umfasst.

9. Pharmazeutische Zusammensetzung, welche mindestens eine Verbindung mit der allgemeinen Formel (I) nach den Ansprüchen 1-8 als Wirkstoff und gegebenenfalls ein oder mehr pharmazeutisch akzeptable(s) Adjuvanz(ien) oder Träger enthält.

10. Verbindung mit der allgemeinen Formel (I) nach den Ansprüchen 1-8 zur Verwendung als Medikament.

11. Verwendung einer Verbindung mit der allgemeinen Formel (I) nach den Ansprüchen 1-8 zur Herstellung eines Medikaments zur Vorbeugung gegen und Behandlung von Augenkrankheiten.

12. Verwendung einer Verbindung mit der allgemeinen Formel (I) nach den Ansprüchen 1-8 zur Herstellung eines Medikaments zur Behandlung von Augenkrankheiten in Verbindung mit anderen Stoffen zur Augenbehandlung.

13. Verfahren zur Synthese von Verbindungen mit der allgemeinen Formel (I) nach den Ansprüchen 1-8, welches mindestens eine Reaktion zwischen dem entsprechenden Ausgangsstoff eines organischen Thiosulfonats oder Dithiol-Thions, Gruppe W oder Y-W, und einer Prostaglandinverbindung oder mindestens eine Reaktion zwischen dem entsprechenden Ausgangsstoff eines organischen Thiosulfonats oder Dithiol-Thions, Gruppe W, und einem entsprechenden Ausgangsstoff eines mit Y modifizierten Prostaglandins umfasst, wobei W und Y die Bedeutung nach Anspruch 1 haben.

## Revendications

1. Composé de formule (I) générale :
A-Y-W (I)
dans laquelle
A est un résidu de prostaglandines ou de leurs dérivés de formule (II) : 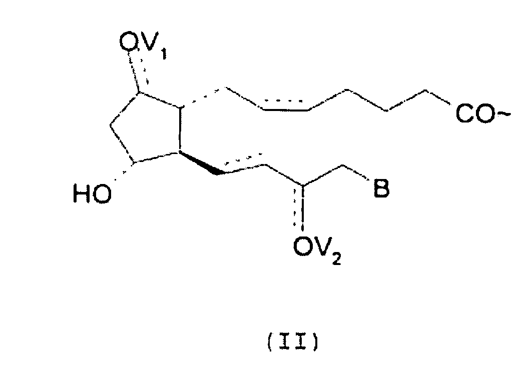 B est -(CH₂)ₘ-CH₃, m est 1 à 5 ; ou 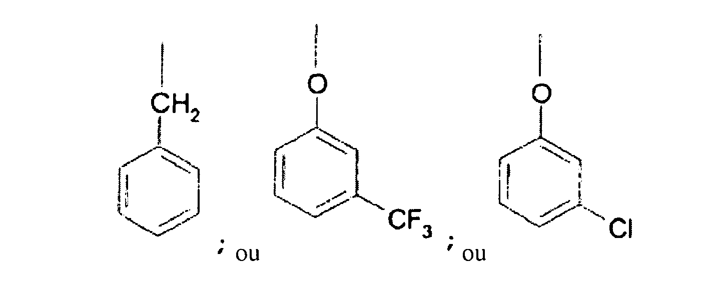 V₁ et V₂, identiques l'un à l'autre ou différents l'un de l'autre, sont zéro ou H ;
la liaison ---- est une simple liaison quand V₁ et/ou V₂ sont H ou une double liaison ;
Y est zéro ; -alkyl en (C_{n'})-, ∼alkyl en (C_{n'})-CO-, ∼O-alkyl en (C_{n'})-O-, ∼OOC-alkyl en (C_{n'})-COO-; ∼O-alkyl en (C_{n'})-, ∼HN-alkyl en (C_{n'})-, ∼OOC-alkyl en (C_{n'})- ; ∼alkyl en (C_{n'})-O-CO-alkyl en (C_{n"})- ; ∼alkyl en (C_{n'})-CO-O-alkyl en (C_{n"})- où l'alkyle en (C_{n'}) et l'alkyle en (C_{n"}) sont linéaires ou ramifiés, et n' et n", identiques l'un à l'autre ou différents l'un de l'autre, sont 0 à 10 ;
W est un groupe polysoufré contenant 2 ou plus de 2 atomes de soufre, choisi dans le groupe comprenant un fragment thiosulfonate organique ou un dérivé de dithiole-thione,
leurs stéréoisomères et l'un de leurs sels.

2. Composés de formule (I) générale selon la revendication 1, dans lesquels W est un fragment thiosulfonate organique de formule :
∼S-SO₂-R (III)
où ∼S-SO₂-R est lié à A-Y~ ; R est un alkyle linéaire ou ramifié, choisi dans le groupe comprenant le méthyle, l'éthyle, le propyle ; un alcényle, un alcynyle ; un alkylaryle, un alcénylaryle, un alcynylaryle ; un arylalkyle, un arylalcényle, un arylalcynyle ou un cycloalkyle, un cycloalcényle, un cycloalcynyle, ou un cycle aromatique et/ou hétérocyclique, tous étant substitués ou non substitués.

3. Composés de formule (I) générale selon la revendication 1, dans lesquels W est un dérivé de dithiole-thione de formule : 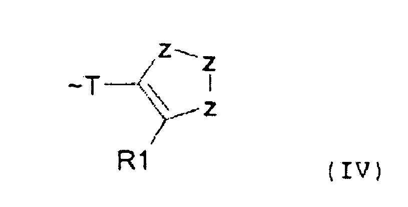 dans laquelle
Z est S (soufre) et au moins 1 Z est une thione C=S ; et T est :
~OOC- ; ou 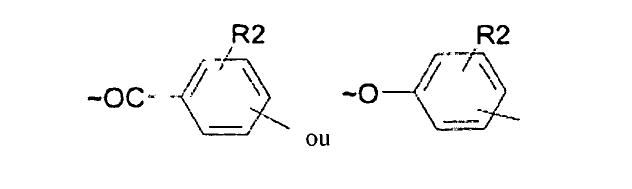 où
R1 est H ; -COOH ; -NH₂ ; -OH ; -SH ;
R2 est un hydrogène ; -COOH ; un alkyle, un alcényle, un alcynyle ; un aryle ; un fluoro, un chloro, un bromo ; un hydroxyle, un alkyloxy, un alcényloxy, un aryloxy, un acyloxy ; un amino, un alkylamino, un arylamino ; un thio ; un cyano ; un nitro ; un acyle ; un amido ; et un cycle aromatique ou non aromatique à 5 ou 6 chaînons contenant 0, 1 ou 2 hétéroatomes choisis parmi l'azote, l'oxygène ou le soufre.

4. Sels de composés de formule (I) générale selon la revendication 1, dans lesquels lesdits sels sont des sels pharmaceutiquement acceptables de composés de formule (I).

5. Sels selon la revendication 4, lesdits sels comprenant des sels de composés de formule (I) avec : des métaux alcalins et des métaux alcalino-terreux, des amines non toxiques et des acides aminés, des acides inorganiques comprenant l'acide chlorhydrique, l'acide phosphorique ou des acides organiques comprenant l'acide fumariques, l'acide citrique, l'acide tartrique.

6. Composés de formule (I) générale selon la revendication 2, dans lesquels le groupe -Y-W est choisi dans le groupe comprenant des fragments de thiosulfonate dérivés des précurseurs correspondants de formule : S-(2-carboxyéthyl)méthanethiosulfonate, S-(2-aminoéthyl)méthanethiosulfonate et S-(2-hydroxyéthyl)méthanethiosulfonate.

7. Composés de formule (I) générale selon la revendication 3, dans lesquels le groupe polysoufré W est choisi dans le groupe comprenant les dérivés de dithiole-thione des précurseurs correspondants de formule : 5-(p-hydroxyphényl)-3H-1,2-dithiol-3-thione, acide 1,3-dithiol-2-thione-5-carboxylique, acide 3-thioxo-3H-1,2-dithiole-5-carboxylique, acide 3-thioxo-3H-1,2-dithiole-4-carboxylique.

8. Composés de formule (I) générale selon la revendication 1, dans lesquels ledit composé est choisi dans le groupe comprenant : l'ester de 4-(3H-1,2-dithiole-3-thione-5-yl)-phényle de l'acide (11a,13*E*,15*S*)-11,15-dihydroxy-9-oxoprost-13-èn-1-oïque, l'ester de 4-(3H-1,2-dithiole-3-thione-5-yl)-phényle de l'acide (5*Z*)-7-[(1*R*,2*R*,3*R*,5*S*)-3,5-dihydroxy-2-[(3*R*)-3-hydroxy-5-phénylpentyl]cyclopentyl]-5-heptènoïque, l'ester de 2-(méthylsulfonylthio)éthyle de l'acide (11α,13*E*,15*S*)-11,15-dihydroxy-9-oxoprost-13-èn-1-oïque, l'ester de 2-(méthylsulfonylthio)éthyle de l'acide (5*Z*)-7-[(1*R*,2*R*,3*R*,5*S*)-3,5-dihydroxy-2-[(3*R*)-3-hydroxy-5-phénylpentyl]-cyclopentyl]-5-heptènoïque, le 2-(méthylsulfonylthio)éthylamide de l'acide (11α,13*E*,15*S*)-11,15-dihydroxy-9-oxoprost-13-èn-1-oïque, le 2-(méthylsulfonyl-thio)éthylamide de l'acide (5*Z*)-7-[(1*R*,2*R*,3*R*,5*S*)-3,5-dihydroxy-2-[(3*R*)-3-hydroxy-5-phénylpentyl]cyclopentyl]-5-heptènoïque, le 2-(méthylsulfonylthio)éthylamide de l'acide (5*Z*,9α,11α,13*E*,15*S*)-9,11,15-trihydroxy-17-phényl-18,19,20-trinorprosta-5,13-diènoïque.

9. Composition pharmaceutique comprenant au moins un composé de formule (I) générale selon les revendications 1 à 8 en tant qu'ingrédient actif et éventuellement un ou plusieurs adjuvant(s) ou support(s) pharmaceutiquement acceptable(s).

10. Composé de formule (I) générale selon les revendications 1 à 8, pour un usage en tant que médicament.

11. Utilisation d'un composé de formule (I) générale selon les revendications 1 à 8, pour la préparation d'un médicament destiné à la prévention, au traitement de maladies oculaires.

12. Utilisation d'un composé de formule (I) générale selon les revendications 1 à 8, pour la préparation d'un médicament destiné au traitement de maladies oculaires, en combinaison avec d'autres agents ophtalmiques.

13. Procédé de synthèse de composés de formule (I) générale selon les revendications 1 à 8, comprenant au moins une réaction entre le précurseur correspondant d'un thiosulfonate organique ou d'une dithiol-thione, un fragment W ou Y-W et un composé de prostaglandine, ou au moins une réaction entre le précurseur correspondant d'un thiosulfonate organique ou d'une dithiol-thione, un fragment W et d'un précurseur correspondant d'une prostaglandine modifiée avec Y, où W et Y ont les définitions données dans la revendication 1.
